# EUROPEAN PATENT SPECIFICATION

(11) **EP 2 274 923 B1**
(45) Date of publication and mention of the grant of the patent: **06.05.2015**
(21) Application number: 08873848.9
(22) Date of filing: 25.08.2008
(51) Int. Cl.: A61N 1/36

(54) **A COCHLEA IMPLANT SYSTEM IN ITE (IN THE EAR) TYPE USING INFRARED DATA COMMUNICATION**
INFRAROT-DATENKOMMUNIKATION VERWENDENDES COCHLEAIMPLANTAT-SYSTEM IM ITE-TYP (IM OHR)
SYSTÈME D'IMPLANT COCHLÉAIRE DANS L'OREILLE (ITE) À L'AIDE D'UNE COMMUNICATION DE DONNÉES PAR INFRAROUGE

(30) Priority: 11.04.2008 KR 20080033760
(43) Date of publication of application: 19.01.2011
(73) Proprietor: M.I.Tech Co., Ltd., Pyeongtaek-si, Gyeonggi-do (KR)
(72) Inventor: KIM, Sung June, Seoul 137-071 (KR); OH, Seung Ha, Seoul 135-284 (KR); AN, Soon Kwan, Seoul 151-055 (KR); LEE, Hong Joo, Goyang-si Gyeonggi-do 410-380 (KR); PARK, Se-ik, Seoul 151-019 (KR)
(74) Representative: Chew, Kwan Chong Daniel
(86) International application number: PCT/KR2008/004951
(87) International publication number: WO 2009/125903

(56) References cited:
- EP-A2- 0 259 906
- WO-A1-98/09588
- WO-A1-99/66982
- WO-A2-02/089913
- KR-A- 20030 036 118
- KR-A- 20030 036 118
- US-A- 4 357 497
- US-A- 5 814 095
- US-A1- 2006 030 905
- US-B1- 6 788 790
- US-B2- 6 537 200
- US-B2- 6 537 200

## Description

### [Technical Field]

The present invention relates to a cochlea implant system including a speech processor and an implanted part, the speech processor and the implanted part communicate via infrared data communication. The implanted part receives its power from the outside through radio-frequency (RF) signal and/or recharges an implantable battery included in the ICS. For this RF power transmission, the speech processor and the implanted part are inductively linked by RF coils.

### [Background Art]

A cochlear implant system is an artificial device for providing auditory sensation by electro-stimulation of remaining auditory neurons of a patient, who is severe-to-profoundly hearing impaired with sensori-neural origins

In general, a cochlea implant comprises a speech processor and an implanted part.

The speech processor provided outside the body processes the acoustic sound (a voice signal or an acoustic signal) received by a microphone and then, transfers the processed signal to the implanted part for the stimulation.

When the signal of the speech processor is received by the implanted part implanted into the body, a stimulation circuit unit converts the received signal into a proper stimulation signal including such information as electrode channel, stimulation mode, magnitude of stimulation, and period of stimulation to be suitable for stimulating the auditory neurons. The stimulation signal is delivered to the auditory neurons via an electrode array inserted into the patient's cochlea. The electrical stimulation is transmitted to the auditory cortex of a brain so that the hearing impaired can hear sounds.

WO 98/09588 A1 discloses a cochlea implant system including an in the ear speech processor and an implanted part. In the cochlea implant system, the speech processor and the implanted part transfer and receive a signal by infrared data communication. The implanted part includes a receiving unit for receiving an infrared signal from the speech processor to demodulate the infrared signal, a stimulation circuit unit for converting the demodulated signal into a stimulation signal, an electrode array inserted into the cochlea to stimulate the auditory neurons by the stimulation signal, and a coil for receiving power from the outside through the RF power transmission. The implanted part can receive power from the outside through the coil to be charged.

EP0259906 A2 discloses a system is described for stimulating a nerve or a muscle fibre of a living body, e.g. a hearing nerve in the cochlea. The system comprises an implant and an electrode for stimulating the nerve or the fibre, which electrode is connected with the implant. To supply electrical power to the implant, a small sized transformer is used, of which one coil is implanted into the body and the other is outside the body but in the vicinity of the implanted coil. In case of the ear, the implanted coil is around the auditory duct and the outer coil is inserted into the duct, so that both coils are substantially coaxial.; For supplying information to the implant, infrared transmission through the skin is used, wherein the transmitter is provided adjacent to the skin on the outside and the receiver, possibly forming a part of the implant is located on the inside of the skin and adjacent thereto.

### [Disclosure]

### [Technical Problem]

The present invention relates to a cochlea implant system including a speech processor and an implanted part that transfer and receive a signal by infrared data communication. The implanted part includes a receiver for receiving an infrared signal from the speech processor to demodulate the infrared signal, a stimulation circuit unit for converting the demodulated signal into a stimulation signal, an electrode array inserted into the cochlea to stimulate the auditory neurons by the stimulation signal, and a coil for receiving power from the outside through radio frequency (RF) power transmission. The implanted part receives power from the outside through the coil to charge an internal battery.

The present invention has been made in view of the above problems, and it is an object of the present invention to provide a cochlea implant system using infrared data communication capable of being charged through the RF power transmission.

### [Technical Solution]

In accordance with an aspect of the present invention, the above and other objects can be accomplished by the provision of a cochlea implant system including a speech processor inserted into the external auditory canal and configured to convert a voice signal or an acoustic signal into an electric signal, to process the electric signal, and to transfer the processed electric signal to an inside of a body, and an implantable part implantable into the mastoid cavity and configured to receive the signal from the speech processor, and to stimulate the auditory neurons in the cochlea. Here, the speech processor and the implantable part transfer and receive the signal by infrared data communication through a skin of the external auditory canal, and
wherein the implantable part comprises a coil implantable under a scalp above a temporal bone mastoid, the coil receiving power from an outside through radio frequency (RF) power transmission through the scalp.

Besides the infrared transmitter for delivering processed signal to the implanted part for stimulation, the speech processor can further include an infrared receiving unit in order to receive information on the status of an electrode array in the body for controlling a proper stimulation range when the cochlea implant system is applied, information on the auditory neural response of a patient in response to a test stimulus, and information on the operation status of the implanted part from the implanted part.

The implanted part includes a receiving unit for receiving an infrared signal from the speech processor to demodulate the infrared signal, a stimulation circuit unit for converting the demodulated signal into a stimulation signal, an electrode array is insertable into the cochlea to stimulate the auditory neurons, and a battery. The implantable part can operate directly by receiving power from the outside through the coil or can charge the battery included in the implantable part using the power received from the outside through the coil. In addition, the implantable part can further include an infrared transmitting unit and can transmit information on the status of an electrode array in the body for controlling a proper stimulation range when the cochlea implant system is applied, information on the auditory neural response of a patient in response to a test stimulus, and information on the operation status of the implantable part to the speech processor through the infrared transmitting unit in accordance with the command signal transmitted by the speech processor.

In addition, there is provided an implantable part implantable into the mastoid cavity to receive a signal from a speech processor and operable to stimulate the auditory neurons in a cochlea implant system. The implantable part includes a receiving unit for receiving an infrared signal from the speech processor through the skin of the external auditory canal to demodulate the infrared signal, a stimulation circuit unit for converting the demodulated signal into a stimulation signal, an electrode array insertable into the cochlea to stimulate the auditory neurons by the stimulation signal, a coil for receiving power from the outside through radio frequency (RF) power transmission, and a battery. In addition, the implantable part according to the present invention can further include a test circuit for extracting information on an electrode array status and a system operation status, a measuring circuit unit for measuring an auditory neural response induced by a test stimulus, and an infrared transmitting unit for transmitting information such as electrode array status, system operation status and auditory neural response to the speech processor.

### [Advantageous Effects]

In the cochlea implant system according to the present invention, the implanted part communicates with the speech processor through the infrared data communication and can receive power from the outside through the coil to be charged.

In particular, according to the present invention, the speech processor is insertable into the external auditory canal and the package of the implantable part is implantable into the mastoid cavity close to the posterior wall of the external auditory canal in order to perform remote infrared data communication with the speech processor insertable into the external auditory cavity.

On the other hand, the implantable part includes the coil so that the implantable part can be charged by the RF power transmission. The coil is implantable under the scalp above the temporal bone mastoid outside the external auditory canal.

Therefore, it is possible to minimize the size of the package implantable into the mastoid cavity of the body.

### [Description of Drawings]

The above and other objects, features and other advantages of the present invention will be more clearly understood from the following detailed description taken in conjunction with the accompanying drawings, in which:
FIG. 1 is a view illustrating that a cochlea implant system according to an embodiment of the present invention;
FIG. 2 is a block diagram of the cochlea implant system according to an embodiment of the present invention;
FIG. 3 is a view illustrating auditory neurons being stimulated by the cochlea implant system according to the present invention;
FIG. 4 is a view illustrating that a cochlea implant system according to another embodiment of the present invention;
FIG. 5 is a block diagram of the cochlea implant system according to another embodiment of the present invention;
FIG. 6 is a view illustrating processes of charging the implantable part of the cochlea implant system according to the present invention;
FIG. 7 is a view illustrating processes of installing a program in the implantable part of the cochlea implant system according to the present invention; and
FIG. 8 is a block diagram of a cochlea implant system capable of performing interactive communications between an implantable part and a speech processor according to still another embodiment of the present invention.

### [Best Mode]

Hereinafter, embodiments of a cochlea implant system according to the present invention will be described in detail with reference to the accompanying drawings. However, the present invention is not limited to the following embodiments.

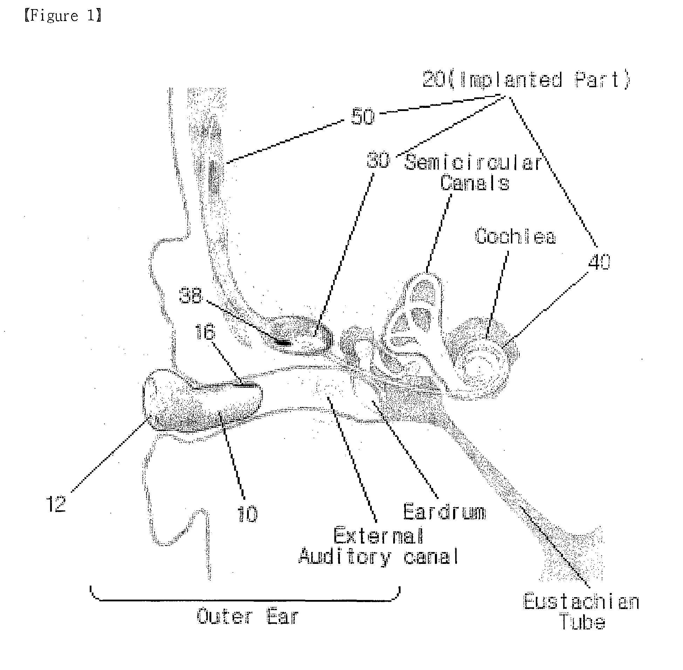

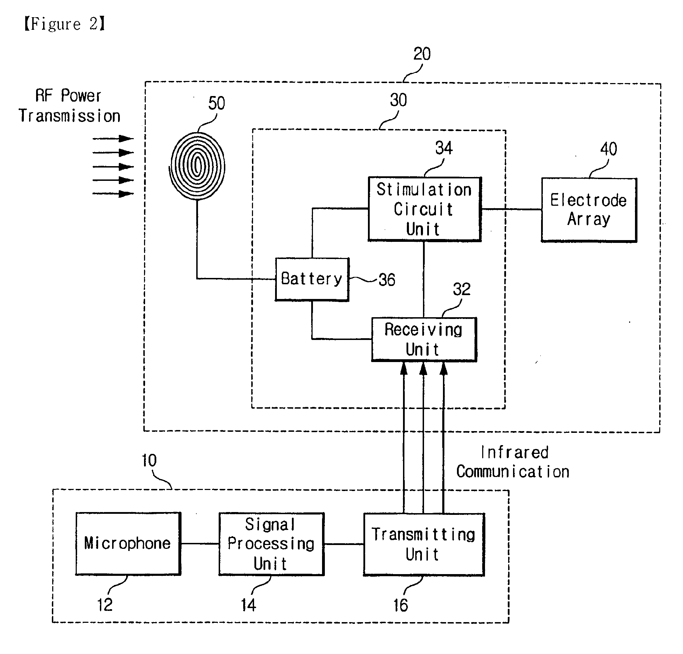

FIG. 1 is a view illustrating that a cochlea implant system according to an embodiment of the present invention. FIG. 2 is a block diagram of the cochlea implant system according to an embodiment of the present invention.

Referring to FIGs. 1 and 2, the cochlea implant system according to the present invention includes a speech processor 10 for converting a voice signal or an acoustic signal into an electric signal, for processing the electric signal, and for transmitting the processed electric signal to the inside of a body and an implanted part 20 for receiving the signal from the speech processor 10 and for stimulating the auditory neurons in the cochlea implant system.

The speech processor 10 and the implanted part 20 can transfer and receive the signal interactively by infrared data communication.

The speech processor 10 includes a microphone 12 for converting the voice signal or the acoustic signal into the electric signal and a transmitting unit 16 for modulating the electric signal, for converting the modulated electric signal into an infrared signal, and for transmitting the converted infrared signal to the inside of the body. The speech processor 10 can further include a signal processing unit 14 for digital signal processing the electric signal of the microphone and a battery (not shown) for driving the speech processor 10.

The speech processor 10 is manufactured to have a proper size so that the speech processor 10 can be inserted into the external auditory canal of a patient. Since the speech processor 10 is inserted into the external auditory canal, it is possible to prevent the patient who uses the cochlea implant system from feeling uncomfortable. In addition, since the microphone 12 is positioned in the external auditory canal, it is possible to minimize the exposure of the cochlea implant system and to maximally utilize the unique functions of the auricle such as the collection of sounds and the sensing of the directions of the sounds.

The transmitting unit 16 includes a modulating unit (not shown) for modulating a signal and a light emitting diode (LED) or a laser diode (LD) for emitting infrared rays in accordance with the modulated signal.

The implanted part 20 includes a receiving unit 32 for receiving an infrared signal from the speech processor 10 and for demodulating the received infrared signal, a stimulation circuit unit 34 for converting the demodulated signal into a stimulation signal, an electrode array 40 inserted into the cochlea to stimulate the auditory neurons by the stimulation signal, a coil 50 for receiving power from the outside through radio frequency (RF) power transmission, and a battery 36.

The receiving unit 32 includes a photo-detector (not shown) for detecting the infrared signal and a demodulating unit (not shown) for demodulating the detected infrared signal.

The receiving unit 32, the stimulation circuit unit 34, and the battery 36 can be provided in a hermetically sealed package 30. The package 30 is preferably formed of a biocompatible material such as metal, ceramic, sapphire glass, aluminum oxide, liquid crystal polymer (LCP), polyimide, biocompatible epoxy, silicone elastomer, or a metal alloy for medical purpose such as stainless steel and titanium alloy, and is hermetically sealed so that body fluids do not permeate into the package 30.

The package 30 of the implanted part 20 is implanted into the mastoid cavity close to the posterior wall of the external auditory canal, in order that the package 30 is implanted as close as possible to the speech processor 10. As such, the speech processor 10 and the implanted part 20 can perform the infrared data communication through the skin of the external auditory canal.

Since the speech processor 10 and the implanted part 20 perform the infrared data communication through the skin of the external auditory canal in the cochlea implant system according to the present invention, it is not necessary to use an external RF coil for transmitting the voice signal or the acoustic signal. Therefore, it is possible to prevent the patient who uses the cochlea implant system from feeling uncomfortable due to the relatively large sized coil.

Therefore, the package 30 is manufactured as small as possible. The package is preferably manufactured so that the width and the height are no more than 20 mm and that the thickness is no more than 10 mm.

An optical window 38 that can transmit the infrared light is provided in the package 30 so that the receiving unit 32 can receive the infrared light from the speech processor 10 and that the transmitting unit 33 can transfer the infrared light. The optical window can be formed of a biocompatible material and that can transmit the infrared light such as sapphire glass, Pyrex glass, biocompatible epoxy, LCP, polyimide, and silicone elastomer.

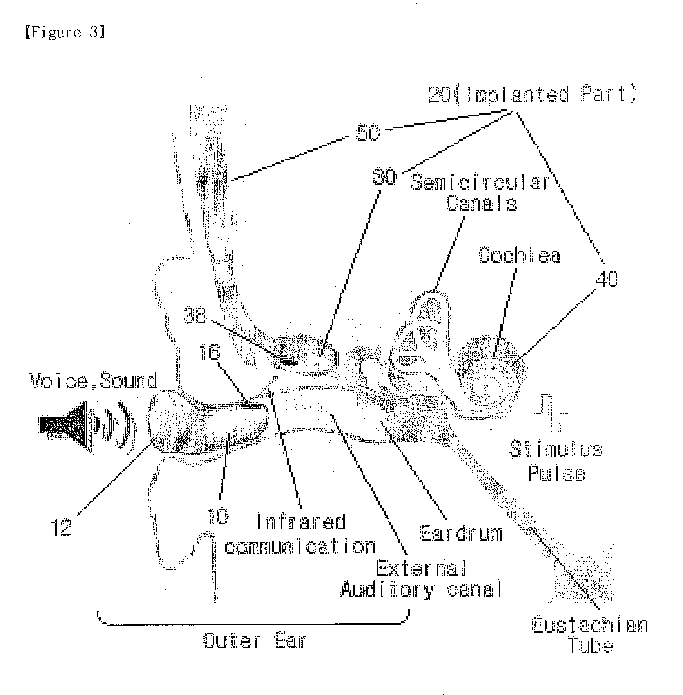

FIG. 3 is a view illustrating that the auditory neurons are stimulated by the cochlea implant system according to the present invention. Referring to FIG. 3, the microphone 12 in the speech processor 10 converts the voice signal or the acoustic signal into the electric signal and the transmitting unit 16 modulates the electric signal, converts the modulated electric signal into the infrared signal, and transfers the infrared signal to the package 30 of the implanted part 20 implanted into the inside of the body.

The receiving unit 32 of the implanted part 20 receives the infrared signal through the optical window 38 and demodulates the received infrared signal. The stimulation circuit unit 34 converts the demodulated signal into a waveform suitable for stimulating the auditory neurons.

The converted signal stimulates the auditory neurons in the cochlea implant system through the electrode array 40 inserted into the cochlea so that the patient can hear external voices or sounds.

In the cochlea implant system according to the present invention, since communications are performed between the speech processor 10 and the implanted part 20 through the infrared signal, a large amount of data can be transferred at high speed, so complicated and various speech processing methods can be applied.

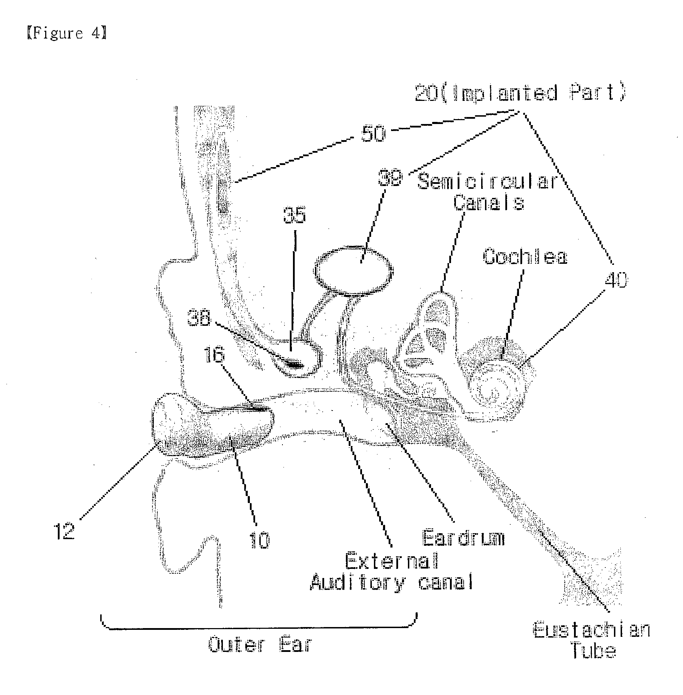

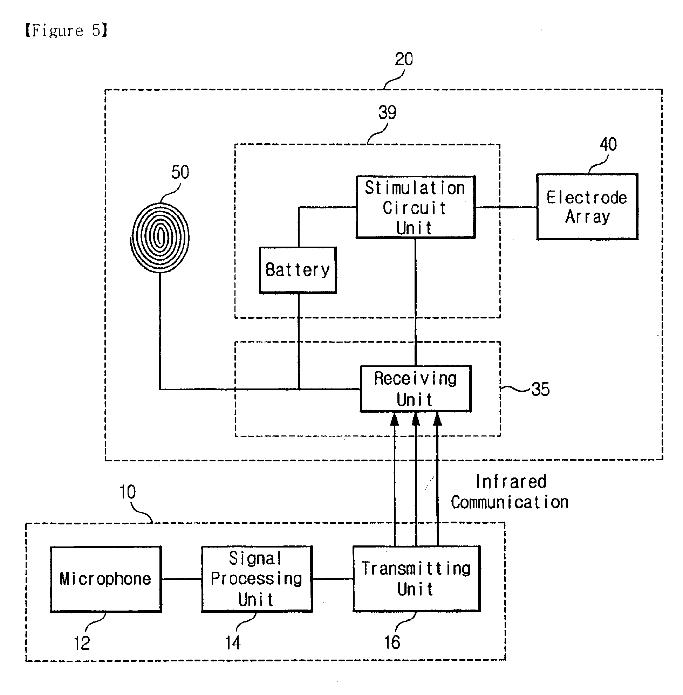

According to the above embodiment, the receiving unit 32, the stimulation circuit unit 34, and the battery are provided in one package 30. However, as an alternative, the receiving unit can be provided in separate package. FIG. 4 is a view illustrating that a cochlea implant system according to another embodiment of the present invention is implanted. FIG. 5 is a block diagram of the cochlea implant system according to another embodiment of the present invention.

In the cochlea implant system according to the present embodiment, as illustrated in FIG. 4, the infrared receiving unit 32 and the optical window 38 that transmits the infrared light are provided in a separate package 35 and the package 35 is implanted into the mastoid cavity. On the other hand, the stimulation circuit unit 34 for converting the signal received through the infrared receiving unit 32 into the stimulation signal and a battery 36 are provided in an additional package 39.

In the above structure, the sizes of the package 35 that performs the infrared data communication are minimized so that the package 35 can be implanted into a more proper position.

The coil 50 for the implanted part 20 receiving power from the outside through RF power transmission is inserted and implanted under the scalp above the temporal bone mastoid close to the mastoid cavity into which the package 30. The battery 36 can be further provided in the package 30 of the implanted part 20 and the battery receives power from the outside through the coil to be charged.

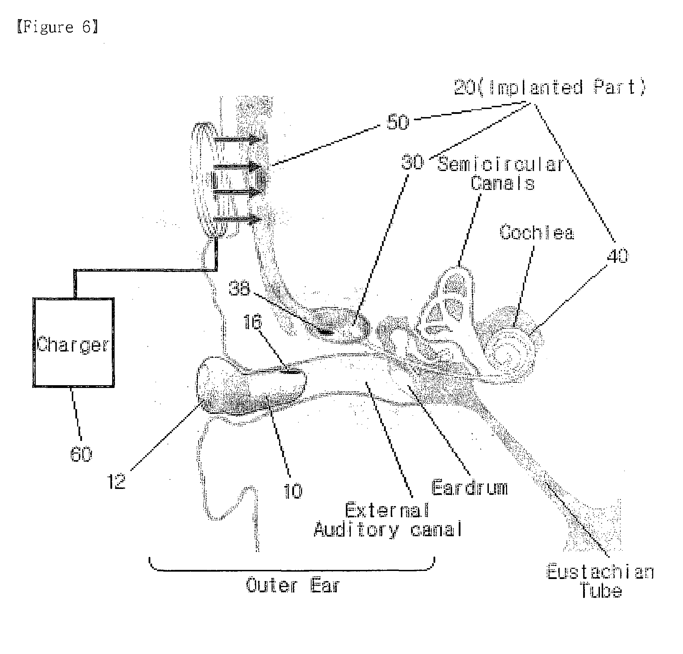

FIG. 6 is a view illustrating processes of supplying power to the implanted part 20 of the cochlea implant system according to the present invention from a charger 60 through the RF power transmission to charge the battery 36 of the implanted part 20. As illustrated in FIG. 6, the implanted part 20 can be charged through the RF power transmission between the coil 62 of the charger 60 and the coil 50 of the implanted part 20. In addition, the implanted part 20 can operate directly by receiving power through the RF power transmission between the external charger 60 including a battery (not shown) and the coil 50 of the implanted part 20.

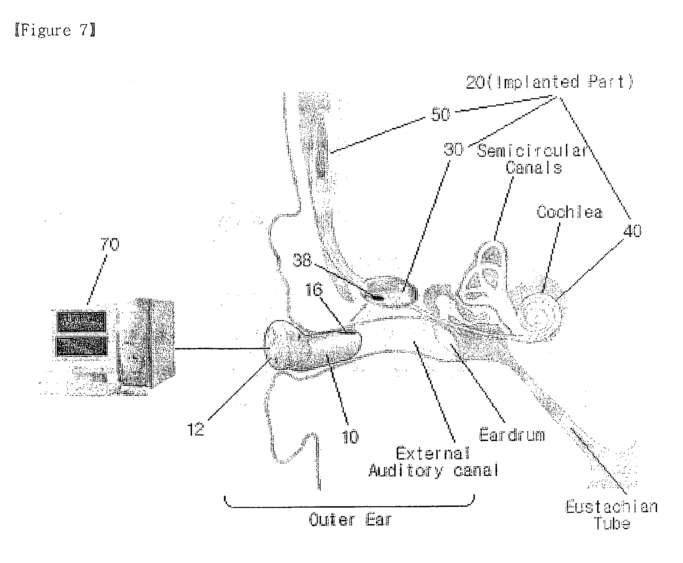

FIG. 7 is a view illustrating processes of connecting a computer 70 to the speech processor 10 to install a program in the implanted part 20 of the cochlea implant system or to upgrade the program according to the present invention. As illustrated in FIG. 7, in order to install or upgrade the program in the implanted part 20, the implanted part 20 and the speech processor 10 must perform interactive communications.

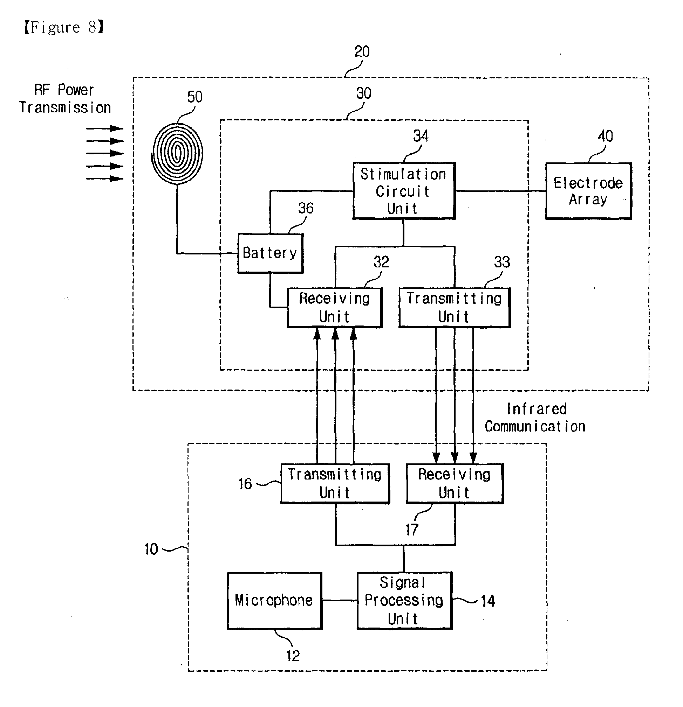

To this end, as illustrated in FIG. 8, the implanted part 20 further includes an infrared transmitting unit 33 capable of transmitting an infrared signal to the speech processor 10 and the speech processor 10 further includes an infrared receiving unit 17 capable of receiving the infrared signal from the implanted part 20. That is, each of the implanted part 20 and the speech processor 10 includes an infrared transmitting unit and an infrared receiving unit.

When the interactive communications can be performed between the implanted part 20 and the speech processor 10, information on the current status of the implanted part 20, for example electrode impedance, can be checked easily.

In particular, the implanted part 20 according to the present invention can further include a test circuit (not shown) in the package 30 for extracting information on an electrode array status and a system operation status and a measuring circuit unit (not shown) for measuring an auditory neural response induced by a stimulation. The information obtained by the test circuit and the measuring circuit unit of the implanted part 20, that is, the electrode array status information, the system operation status information, and the auditory neural response information, are modulated to a form suitable for the infrared data communication, the modulated signal is transmitted to the speech processor through the infrared transmitting unit, and the information is transmitted to the computer 70.

### [Industrial Applicability]

In the cochlea implant system according to the present invention, the implanted part communicates with the speech processor through the infrared data communication and receives power from the outside through the coil to be operated or to be charged.

In particular, according to the present invention, the speech processor is insertable into the external auditory canal and the package of the implantable part is implantable into the mastoid cavity close to the posterior wall of the external auditory canal in order to perform the remote infrared data communication with the speech processor inserted into the external auditory canal.

On the other hand, the implantable part includes the coil so that the implantable part can be charged by the RF power transmission. The coil is implantable under the scalp above the temporal bone mastoid outside the external auditory canal.

Therefore, it is possible to minimize the size of the package implantable into the mastoid cavity of the body.

In addition, the function of a hearing aid can be combined with the cochlea implant system according to the present invention.

Although the preferred embodiments of the present invention have been disclosed for illustrative purposes, those skilled in the art will appreciate that various modifications, additions and substitutions are possible.

## Claims

1. A cochlea implant system, comprising:
a speech processor (10) configured to be inserted into the external auditory canal, to convert a voice signal or an acoustic signal into an electric signal, to process the electric signal, and to transfer the processed electric signal to an inside of a body; and
an implantable part (20) implantable into the mastoid cavity and configured to receive the signal from the speech processor, and to stimulate the auditory neurons in a cochlea.
wherein the speech processor and the implantable part are configured to transfer and receive the signal by infrared data communication through a skin of the external auditory canal, and
wherein the implantable part comprises a coil (50) implantable under a scalp above a temporal bone mastoid, the coil configured for receiving power from an outside through radio frequency (RF) power transmission through the scalp.

2. The cochlea implant system as set forth in claim 1, wherein the implantable part (20) further comprises:
a receiving unit (32) for receiving an infrared signal from the speech processor (10) to demodulate the infrared signal;
a stimulation circuit unit (34) for converting the demodulated signal into a stimulation signal; and
an electrode array (40) configured to be inserted into the cochlea to stimulate the auditory neurons by the stimulation signal.

3. The cochlea implant system as set forth in claim 2,
further comprising a package (30), for hermetically sealing the receiving unit (32) and the stimulation circuit unit (34), wherein the package is manufactured so that a width and a height of the package are no more than 20mm and a thickness of the package is no more than 10mm.

4. The cochlea implant system as set forth in claim 3, wherein the package (30) is formed of a biocomptable material such as metal, ceramic, sapphire glass, aluminum oxide, liquid crystal polymer (LCP), polyimide, biocompatible epoxy, silicone elastomer, stainless steel, or titanium alloy, wherein an optical window that transmits infrared light may be provided in the package and, in this case, the optical window may be formed of a bicompatible material such as sapphire glass, Pyrex glass, biocompatible epoxy, LCP, polymide, or silicone elastomer.

5. The cochlea implant system as set forth in any of claims 2 to 4,
wherein the implantable part (20) further comprises a battery (36), and
wherein the implantable part is configured to receive power from the outside through the coil (50) and to charge the battery, wherein the implantable part may be configured to receive power from the outside through the coil to operate.

6. The cochlea implant system as set forth in claim 2,
further comprising packages (35, 39) for separately hermetically sealing the receiving unit and the stimulation circuit unit (34), respectively, and
wherein the package (35) in which the receiving unit (32) is provided comprises an optical window that can transmit infrared rays.

7. The cochlea implant system as set forth in claim 1, further comprising:
a test circuit for extracting information on an electrode array (40) status and a system operation status; and
a measuring circuit unit for measuring an auditory neural response signal induced by a test stimulus, wherein the implantable part (20) further comprises an infrared transmitting unit for transmitting electrode array status information, system operation status information, and auditory neural response information obtained by the test circuit and the measuring circuit unit to the speech processor in the form of an infrared signal.

8. The cochlea implant system as set forth in any of claims 2 to 6, wherein the receiving unit (32) comprises a photo-detector for detecting the infrared signal and a demodulating unit for demodulating the detected infrared signal.

9. The cochlea implant system as set forth in any of claims 1 to 8, wherein the speech processor comprises:
a microphone (12) for converting the voice signal or the acoustic signal into an electric signal; and
a transmitting unit (16) for modulating the electric signal, for converting the modulated electric signal into an infrared signal, and for transmitting the converted infrared signal to the inside of the body.

10. The cochlea implant system as set forth in claim 9, wherein the speech processor (10) further comprises a receiving unit (17) for receiving the infrared signal from the implantable part (20), wherein the transmitting unit may comprise:
a modulating unit for modulating a signal; and
a light emitting diode (LED) or a laser diode (LD) for emitting infrared light in accordance with the modulated signal.

## Patentansprüche

1. Cochleaimplantatsystem, das aufweist:
einen Sprachprozessor (10), der ausgelegt ist, in den externen Hörkanal eingeführt zu werden, ein Sprachsignal oder ein akustisches Signal in ein elektrisches Signal umzuwandeln, das elektrische Signal zu verarbeiten und das verarbeitete elektrische Signal in ein Inneres eines Körpers zu übertragen; und
einen implantierbaren Teil (20), der in die Mastoidhöhle implantierbar ist und ausgelegt ist, das Signal von dem Sprachprozessor zu empfangen und die Hörnerven in einer Cochlea zu stimulieren,
wobei der Sprachprozessor und der implantierbare Teil ausgelegt sind, das Signal mittels Infrarotdatenkommunikation durch eine Haut des externen Hörkanals zu übertragen und empfangen, und
wobei der implantierbare Teil eine Spule (50) aufweist, die unter eine Kopfhaut oberhalb eines Schläfenbein-Mastoids implantierbar ist, wobei die Spule zum Empfangen von Energie von einer Außenseite mittels Radiofrequenzenergieübertragung (RF) durch die Kopfhaut ausgelegt ist.

2. Cochleaimplantatsystem nach Anspruch 1, wobei der implantierbare Teil (20) außerdem aufweist:
eine Empfangseinheit (32) zum Empfangen eines Infrarotsignals von dem Sprachprozessor (10), um das Infrarotsignal zu demodulieren;
eine Stimulationsschaltungseinheit (34) zum Umwandeln des demodulierten Signals in ein Stimulierungssignal; und
ein Elektroden-Array (40), das ausgelegt ist, in die Cochlea eingeführt zu werden, um die Hörnerven durch das Stimulierungssignal zu stimulieren.

3. Cochleaimplantatsystem nach Anspruch 2,
das außerdem ein Gehäuse (30) zum hermetischen Abdichten der Empfangseinheit (32) und der Stimulationsschaltungseinheit (34) aufweist, wobei das Gehäuse derart hergestellt ist, dass eine Breite und eine Höhe des Gehäuses nicht größer als 20mm sind und eine Dicke des Gehäuses nicht größer als 10mm ist.

4. Cochleaimplantatsystem nach Anspruch 3, wobei das Gehäuse (30) aus einem biokompatiblen Material wie Metall, Keramik, Saphirglas, Aluminiumoxid, Flüssigkristallpolymer (LCP), Polyimid, biokompatibles Epoxyd, Silikonelastomer, rostfreier Stahl oder Titanlegierung ausgebildet ist, wobei ein optisches Fenster, das Infrarotlicht durchlässt, in dem Gehäuse angeordnet sein kann und in diesem Fall das optische Fenster aus einem biokompatiblen Material wie Saphirglas, Pyrex-Glas, biokompatibles Epoxyd, LCP, Polyimid oder Silikonelastomer ausgebildet sein kann.

5. Cochleaimplantatsystem nach einem der Ansprüche 2 bis 4,
wobei der implantierbare Teil (20) außerdem eine Batterie (36) aufweist, und
wobei der implantierbare Teil ausgelegt ist, Energie von der Außenseite durch die Spule (50) zu empfangen und die Batterie zu laden, wobei der implantierbare Teil ausgelegt sein kann, Energie von der Außenseite durch die Spule für einen Betrieb zu empfangen.

6. Cochleaimplantatsystem nach Anspruch 2,
das außerdem Gehäuse (35, 39) zum getrennten hermetischen Abdichten der Empfangseinheit und der Stimulationsschaltungseinheit (34) aufweist, und
wobei das Gehäuse (35), in dem die Empfangseinheit (32) angeordnet ist, ein optisches Fenster aufweist, das Infrarotstrahlen durchlassen kann.

7. Cochleaimplantatsystem nach Anspruch 1, das außerdem aufweist:
eine Testschaltung zum Extrahieren von Informationen über einen Status eines Elektroden-Arrays (40) und einen Systembetriebsstatus; und
eine Messschaltungseinheit zum Messen eines Hörnervenantwortsignals, das durch einen Testreiz herbeigeführt wird, wobei der implantierbare Teil (20) außerdem eine Infrarotsendeeinheit zum Senden von Elektroden-Arraystatusinformationen, Systembetriebsstatusinformationen und Hörnervenantwortinformationen, die von der Testschaltung und der Messschaltungseinheit erhalten werden, an den Sprachprozessor in der Form eines Infrarotsignals aufweist.

8. Cochleaimplantatsystem nach einem der Ansprüche 2 bis 6, wobei die Empfangseinheit (32) einen Photodetektor zum Erfassen des Infrarotsignals und eine Demoduliereinheit zum Demodulieren des erfassten Infrarotsignals aufweist.

9. Cochleaimplantatsystem nach einem der Ansprüche 1 bis 8, wobei der Sprachprozessor aufweist:
ein Mikrophon (12) zum Umwandeln des Sprachsignals oder akustischen Signals in ein elektrisches Signal; und
eine Sendeeinheit (16) zum Modulieren des elektrischen Signals, zum Umwandeln des modulierten elektrischen Signals in ein Infrarotsignal und zum Senden des umgewandelten Infrarotsignals in das Innere des Körpers.

10. Cochleaimplantatsystem nach Anspruch 9, wobei der Sprachprozessor (10) außerdem eine Empfangseinheit (17) zum Empfangen des Infrarotsignals von dem implantierbaren Teil (20) aufweist, wobei die Sendeeinheit aufweisen kann:
eine Moduliereinheit zum Modulieren eines Signals; und
eine lichtemittierende Diode (LED) oder eine Laserdiode (LD) zum Emittieren von Infrarotlicht entsprechend dem modulierten Signal.

## Revendications

1. Système d'implant cochléaire, comprenant :
un processeur de parole (10) configuré pour être inséré dans le canal auditif externe, pour convertir un signal vocal ou un signal acoustique en un signal électrique, pour traiter le signal électrique, et pour transférer le signal électrique traité vers un intérieur d'un corps ; et
un élément implantable (20) implantable dans la cavité mastoïdienne et configuré pour recevoir le signal en provenance du processeur de parole, et pour stimuler les neurones auditifs dans une cochlée,
dans lequel le processeur de parole et l'élément implantable sont configurés pour transférer et recevoir le signal par communication de données par infrarouge à travers une paroi du canal auditif externe, et
dans lequel l'élément implantable comprend une bobine (50) implantable sous un cuir chevelu au-dessus d'un mastoïde de l'os temporal, la bobine étant configurée pour recevoir une alimentation depuis un extérieur par le biais d'une transmission de puissance radiofréquence (RF) à travers le cuir chevelu.

2. Système d'implant cochléaire selon la revendication 1, dans lequel l'élément implantable (20) comprend en outre :
une unité de réception (32) pour recevoir un signal infrarouge du processeur de parole (10) pour démoduler le signal infrarouge ;
une unité de circuit de stimulation (34) pour convertir le signal démodulé en un signal de stimulation ; et
un réseau d'électrodes (40) configuré pour être inséré dans la cochlée pour stimuler les neurones auditifs au moyen du signal de stimulation.

3. Système d'implant cochléaire selon la revendication 2,
comprenant en outre un boitier (30) pour enfermer hermétiquement l'unité de réception (32) et l'unité de circuit de stimulation (34), dans lequel le boîtier est fabriqué de telle sorte qu'une largeur et une hauteur du boîtier ne dépassent pas 20 mm et qu'une épaisseur du boîtier ne dépasse pas 10 mm.

4. Système d'implant cochléaire selon la revendication 3, dans lequel le boîtier (30) est constitué d'un matériau biocompatible tel que du métal, de la céramique, du verre saphir, de l'oxyde d'aluminium, du polymère à cristaux liquides (PCL), du polyimide, de l'époxy biocompatible, de l'élastomère de silicone, de l'acier inoxydable ou de l'alliage de titane, dans lequel une fenêtre optique qui transmet la lumière infrarouge peut être prévue dans le boîtier et, dans ce cas, la fenêtre optique peut être constituée d'un matériau biocompatible tel que du verre saphir, du verre Pyrex, de l'époxy biocompatible, du PCL, du polyimide ou de l'élastomère de silicone.

5. Système d'implant cochléaire selon l'une quelconque des revendications 2 à 4,
dans lequel l'élément implantable (20) comprend en outre une batterie (36), et
dans lequel l'élément implantable est configuré pour recevoir de l'énergie depuis l'extérieur à travers la bobine (50) et pour charger la batterie,
dans lequel l'élément implantable peut être configuré pour recevoir de l'énergie depuis l'extérieur à travers la bobine pour fonctionner.

6. Système d'implant cochléaire selon la revendication 2,
comprenant en outre des boîtiers (35, 39) pour enfermer hermétiquement de manière séparée l'unité de réception et l'unité de circuit de stimulation (34), respectivement, et
dans lequel le boîtier (35) dans lequel est prévue l'unité de réception (32) comprend une fenêtre optique qui peut transmettre les rayons infrarouges.

7. Système d'implant cochléaire selon la revendication 1, comprenant en outre :
un circuit de test pour extraire des informations sur un état de réseau d'électrodes (40) et un état de fonctionnement du système ; et
une unité de circuit de mesure pour mesurer un signal de réponse neuronale auditive provoqué par un stimulus de test, dans lequel l'élément implantable (20) comprend en outre une unité de transmission infrarouge pour transmettre des informations d'état de réseau d'électrodes, des informations d'état de fonctionnement du système et des informations de réponse neuronale auditive obtenues par le circuit de test et
l'unité de circuit de mesure au processeur de parole sous la forme d'un signal infrarouge.

8. Système d'implant cochléaire selon l'une quelconque des revendications 2 à 6, dans lequel l'unité de réception (32) comprend un photo-détecteur pour détecter le signal infrarouge et une unité de démodulation pour démoduler le signal infrarouge détecté.

9. Système d'implant cochléaire selon l'une quelconque des revendications 1 à 8, dans lequel le processeur de parole comprend :
un microphone (12) pour convertir le signal vocal ou le signal acoustique en un signal électrique ; et
une unité de transmission (16) pour moduler le signal électrique, pour convertir le signal électrique modulé en un signal infrarouge, et pour transmettre le signal infrarouge converti à l'intérieur du corps.

10. Système d'implant cochléaire selon la revendication 9, dans lequel le processeur de parole (10) comprend en outre une unité de réception (17) pour recevoir le signal infrarouge à partir de l'élément implantable (20), dans lequel l'unité de transmission peut comprendre :
une unité de modulation pour moduler un signal ; et
une diode électroluminescente (DEL) ou une diode laser (DL) pour émettre une lumière infrarouge selon le signal modulé.
